# EUROPÄISCHE PATENTANMELDUNG

(11) **EP 1 803 703 A1**
(43) Veröffentlichungstag der Anmeldung: **04.07.2007**
(21) Anmeldenummer: 05113051.6
(22) Anmeldetag: 29.12.2005
(51) Int. Cl.: C07C 45/78

(54) **Verfahren zur Reinigung von Alkenonethern durch Ausfrieren**

(71) Anmelder: Solvay Fluor GmbH, 30173 Hannover (DE)
(72) Erfinder: Wiesenhöfer, Wolfgang, 40885 Ratingen (DE); Kalbreyer, Wolfgang, 31595, Steyerberg (DE); Eicher, Johannes, 31319 Sehnde (DE)
(74) Vertreter: Jacques, Philippe

(57) **Zusammenfassung**

Halogenalkenonether sind Zwischenprodukte in der chemischen Synthese. Bei der Lagerung entstehen Nebenprodukte, die durch Ausfrieren entfernt werden können.

## Beschreibung

Die Erfindung bezieht sich auf ein Verfahren zur Reinigung von halogenierten Alkenonethern.

Halogenierte Alkenonether, beispielsweise 4-Ethoxy-1,1,1-trifluoro-3-buten-2-on ("ETFBO"), sind Bausteine in der chemischen Synthese, siehe beispielsweise EP-0 744 400. Man kann sie herstellen, indem man ein Säurechlorid mit einem Vinylether in Anwesenheit einer Base miteinander umsetzt, siehe die obengenannte europäische Offenlegungsschrift; dabei kann die Base auch im Überschuss als Lösemittel eingesetzt werden.

Die WO 03/080562 offenbart ein Verfahren zur Herstellung halogenierten Alkenonethern aus Säurechloriden und Vinylethern in Anwesenheit von Oniumsalzen. Die W02004/108647 offenbart die Herstellung aus Säurechloriden und Vinylethern in Abwesenheit von Basen oder Oniumsalzen bzw. in der Anwesenheit von Stabilisatoren.

Es hat sich gezeigt, dass - gemäß NMR- und GC-(gaschromatographischen) Untersuchungen - sich in halogenhaltigen acylsubstituierten Alkenonethern beim Stehen Verunreinigungen bilden, bei denen es sich möglicherweise um Dimere handelt, die sich in einer Hetero-Diels-Alder-Reaktion bilden. ETFBO ist beispielsweise nicht unempfindlich gegenüber Feuchtigkeit in der umgebenden Luft, mit welcher ETFBO beim Abfüllen oder Umfüllen oder bei unsachgemäßer Lagerung in Kontakt kommen kann. Die Folgeprodukte dieser Hydrolysereaktion sind unter anderem Aldehyde und Alkohole; die letzteren reagieren ihrerseits mit den halogenierten Alkenonethern in einer Additionsreaktion. Der oder die gebildeten Aldehyde oligomerisieren auf verschiedene Weise, z.B. in einer Aldolreaktion, zu einer Vielzahl von Nebenprodukten. Aufgabe der vorliegenden Erfindung war es, ein einfaches Verfahren anzugeben, mit welchem sich solche Verunreinigungen abtrennen lassen.

Diese Aufgabe wird durch das Verfahren der vorliegenden Erfindung gelöst.

Das erfindungsgemäße Verfahren zur Reinigung von halogensubstituierten acylierten Alkenonethern sieht vor, dass man den Alkenonether abkühlt, so dass sich eine feste Phase, die den gereinigten halogenierten Alkenonether darstellt, und eine flüssige Phase bilden, man die feste Phase von der flüssigen Phase abtrennt und so den gereinigten Alkenonether gewinnt.

Bevorzugt ist ein Verfahren zur Reinigung von Alkenonethern mit Fluor- und/oder Chlorsubstitution am Carbonsäurerest. Ganz besonders bevorzugt reinigt man Alkenonether der Formel (I)

R¹-C(O)-C(H)=C(H)-OR² (I),

wobei R¹ für eine C1-C4-Alkylgruppe oder für eine C1-C4-Alkylgruppe steht, die durch mindestens 1 Halogenatom substituiert ist, oder wobei R¹ für CF₃C(O)CH₂ steht, und wobei R² für Aryl, substituiertes Aryl, eine C1-C4-Alkylgruppe oder für eine C1-C4-Alkylgruppe steht, die durch mindestens 1 Halogenatom substituiert ist. Solche Verbindungen können gemäß der Lehre der WO 03/080562 oder WO2004/108647 hergestellt werden, indem man ein Säurehalogenid der Formel (II)

R¹-C(O)X (II),

worin X für F, Cl oder Br steht und R¹ obengenannte Bedeutung besitzt, mit einem Vinylether der Formel (III)

CH₂ = C(H)-OR² (III),

worin R² die obengenannte Bedeutung besitzt, miteinander umsetzt. Dies kann, wie aus dem Stand der Technik bekannt, in Anwesenheit einer Base, in Anwesenheit eines Oniumsalzes oder Abwesenheit eines Säurefängers und/oder in Anwesenheit eines Stabilisators für den Alkenonether geschehen.

R¹ steht bevorzugt für Methyl, Ethyl, n-Propyl oder i-Propyl oder durch mindestens 1 Fluoratom substituiertes Methyl, Ethyl, n-Propyl oder i-Propyl. Besonders bevorzugt steht R¹ für Methyl, Ethyl oder durch mindestens 1 Fluoratom substituiertes Methyl oder Ethyl. Ganz besonders bevorzugt steht R¹ für CF₃, CF₂H, CF₂Cl, C₂F₅, C₃F₇ oder CF₃C(O)CH₂.

R² kann für Aryl, beispielsweise Phenyl oder C1-C4-Alkylgruppen und/oder Halogenatome substituiertes Phenyl stehen. Bevorzugt bedeutet R² lineares oder verzweigtes C1-C4-Alkyl. Ganz besonders bevorzugt bedeutet R² Methyl, Ethyl, n-Propyl oder i-Propyl.

Die Alkenonetherverbindungen können als cis- oder trans-Isomere oder als Gemische von cis- und trans-Isomer vorliegen und gemäß der Erfindung gereinigt werden.

Die zu reinigende Alkenonetherverbindung wird auf eine Temperatur abgekühlt, die sich nach der Art und dem Grad der Verunreinigung richtet. Sie liegt unterhalb des Festpunktes der Alkenonether-Verbindung. Je nach Verbindung ist ein Abkühlen auf eine Temperatur unterhalb von -10°C bevorzugt. Die Untergrenze wird durch den Festpunkt der Verunreinigungen bestimmt. Meist ist eine Abkühlung auf eine Temperatur im Bereich von - 25 bis - 80 °C gut geeignet, eine Temperatur im Bereich von -30 bis -45 °C sehr gut geeignet und eine Temperatur von -33 bis -37 °C besonders gut geeignet, um Verunreinigungen abtrennen zu können.

Die Abtrennung des gebildeten Feststoffes von der Flüssigkeit ist durch Dekantieren oder Filtern möglich; dabei können Apparaturen von Vorteil sein, die ebenfalls gekühlt werden können. Die abgetrennte Flüssigkeit mit den Ver-unreinigungen enthält noch größere Anteile des Alkenonethers, so dass es sich lohnen kann, die Flüssigkeit nicht zu verwerfen, sondern sie aufzuarbeiten oder wieder-zuverwerten, beipielsweise, indem man sie einer späteren Charge zusetzt und destilliert.

Vorteil des erfindungsgemäßen Verfahrens ist, dass die Abtrennung von Verunreinigungen durch eine einfache Verfahrensoperation - Abkühlen und Trennen von Feststoff und Flüssigkeit - bewirkt werden kann.

Die folgenden Beispiele sollen die Erfindung weiter erläutern, ohne sie in ihrem Umfang einzuschränken.

### Beispiele

### Beispiel: Reinigung von 4-Ethoxy-1,1,1-trifluoro-3-buten-2-on (ETFBO)

### Durchführung:

7.7 kg "gealtertes" ETFBO mit einem Gehalt von 96.1 % ETFBO (cis + trans-Isomer) wurden bei -35 °C für 48 Stunden gelagert. Dabei kristallisierte ein weißer Feststoff aus, und eine Flüssigkeit blieb zurück. Der Feststoff wurde durch einfaches Dekantieren von der Flüssigkeit getrennt. Man erhielt zwei Chargen ETFBO mit unterschiedlicher Reinheit. Die bei -35 °C flüssige Charge (3.6 kg) hat einen ETFBO-Gehalt von 91.6 % (cis+trans). Die bei -35 °C feste Charge (4.1 kg) hat einen ETFBO-Gehalt von 99.0 % (cis+trans). Die Ergebnisse werden in Tabelle 1 zusammengefasst:

**Tabelle 1**

| Probe | "gealtertes" ETFBO vor dem Einfrieren | flüssige Charge | feste Charge |
|---|---|---|---|
| Menge | 7.7 kg | 3.6 kg | 4.1 kg |
| GC-Gehalt trans-ETFBO | 94.5 % | 90.4 % | 97.7 % |
| GC-Gehalt cis ETFBO | 1.6 % | 1.2 % | 1.2 % |
| GC-Gehalt DE-ETFBO | 2.0 | 3.7 | 0.5 |
| GC-Gehalt Dimer | 0.4 | 1.2 | 0.2 |
| Signalanzahl im GC | 30 | 21 | 11 |
| Summe cis + trans | 96.1 % | 91.6 % | 99.0 % |

### Beispiel 2

### Beispiel: Reinigung von 4-Ethoxy-1,1,1-trifluoro-3-buten-2-on (ETFBO)

Durchführung:
Analog Beispiel 1 wurden 4.2 kg ETFBO mit einem Gehalt von 96.6 % ETFBO (cis + trans-Isomer) aufgereinigt. Die Ergebnisse sind in Tabelle 2 zusammengefaßt dargestellt.

**Tabelle 2**

| Probe | "gealtertes" ETFBO vor dem Einfrieren | flüssige Charge | feste Charge |
|---|---|---|---|
| Menge | 4.2 kg | 1.6 | 2.6 |
| GC-Gehalt trans-ETFBO | 95.1 | 93.9 | 97.0 |
| GC-Gehalt cis ETFBO | 1.7 | 1.8 | 1.5 |
| GC-Gehalt DE-ETFBO | 0.9 | 0.8 | 0.8 |
| GC-Gehalt Dimer | 0.1 | 0.4 | < 0.1 |
| Signalanzahl im GC | 18 | 16 | 15 |
| Summe cis + trans | 96.6 | 95.7 | 98.5 |

Die beiden Beispiele belegen, dass durch einfaches Ausfrieren eine sehr effektive Reinigung von ETFBO möglich ist.

## Patentansprüche

1. Verfahren zur Herstellung von gereinigten Alkenonethern, wobei man die Alkenonether abkühlt, so dass der Alkenonether ausfällt und verunreinigende Bestandteile flüssig bleiben, und man die Verunreinigungen vom Alkenonether abtrennt.

2. Verfahren nach Anspruch 1, **dadurch gekennzeichnet, dass** man ein Alkenon der Formel (I) reinigt
R¹-C(O)-C(H)=C(H)-OR² (I)
wobei R¹ für eine C1-C4-Alkylgruppe oder für eine C1-C4-Alkylgruppe steht, die durch mindestens ein Halogenatom substituiert ist, oder wobei R¹ für CF₃C(O)CH₂ steht und R² für Aryl, substituiertes Aryl, eine C1-C4-Alkylgruppe oder für eine C1-C4-Alkylgruppe steht, die durch mindestens ein Halogenatom substituiert ist.

3. Verfahren nach Anspruch 1, **dadurch gekennzeichnet, dass** R¹ für Methyl, Ethyl oder Propyl oder durch mindestens 1 Fluoratom substituiertes Methyl, Ethyl oder Propyl steht.

4. Verfahren nach Anspruch 1, **dadurch gekennzeichnet, dass** R¹ für CF₃, CF₂H, CF₂Cl, C₂F₅, C₃F₇ oder CF₃C(O)CH₂ steht.

5. Verfahren nach Anspruch 1, **dadurch gekennzeichnet, dass** R² für Methyl, Ethyl, n-Propyl oder iso-Propyl steht.

6. Verfahren nach Anspruch 1, **dadurch gekennzeichnet, dass** man den zu reinigenden Alkenonether auf eine Temperatur von -25 bis -80 °C abkühlt.

7. Verfahren nach Anspruch 6, **dadurch gekennzeichnet, dass** man den zu reinigenden Alkenonether auf eine Temperatur von -30 bis -45 °C abkühlt.

8. Verfahren nach Anspruch 1, **dadurch gekennzeichnet, dass** man den zu reinigenden Alkenonether auf eine Temperatur von -33 bis -37 °C abkühlt, insbesondere auf eine Temperatur von -35 °C.
